(19) Europäisches Patentamt
European Patent Office
Office européen des brevets

(11) EP 3 379 381 A1

(12) EUROPEAN PATENT APPLICATION

(43) Date of publication:
26.09.2018 Bulletin 2018/39

(51) Int Cl.:
G06F 3/01 (2006.01)     A61H 19/00 (2006.01)
G06F 3/0346 (2013.01)

(21) Application number: 17176367.5

(22) Date of filing: 16.06.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 23.03.2017 CN 201710178896

(71) Applicant: Virtual Matrix Technologies Inc.
Arcadia, CA 91007 (US)

(72) Inventors:
• CHEN, Conghui
TIANCHANG City, Anhui (CN)
• HU, Hui
Suzhou City, Jiangsu (CN)

(74) Representative: Casalonga
Casalonga & Partners
Bayerstraße 71/73
80335 München (DE)

(54) **METHOD AND SYSTEM FOR INTERACTING WITH INTELLIGENT ADULT PRODUCT**

(57)     The present invention relates a method and system for interacting with an intelligent adult product. The method comprises the steps of: establishing connection between an intelligent adult product and an application in a user's mobile terminal; controlling, by an acceleration sensor in the intelligent adult product, the motion of a three-dimensional character in the application; and, viewing, by a user and by using a VR box, sounding persons and objects in a virtual scene in a first-person perspective or a third-person perspective. In the technical solutions provided by the present invention, by allowing a user to view a virtual and vivid character scene by wearing a VR box and then controlling the motion of characters in the virtual scene by an intelligent masturbation cup or an intelligent bracelet, the motion synchronization between a real character and a virtual character is realized. Thus, the user is provided with visually and audibly immersive experience, and also most exciting tactile experience.

VR box

Intelligent bracelet

Application

Intelligent masturbation cup

Fig 1

**Description**

**TECHNICAL FIELD**

The present invention relates to the field of information communication, and in particular to a method and system for interacting with an intelligent adult product.

**BACKGROUND OF THE PRESENT INVENTION**

With the improvement of social civilization, the people's attitude to "sex" changes from unmentionable to unavoidable, and people are increasingly paying attention to correctly dealing with the physiological needs. When realistic problems such as bachelordom and long-distance separation seriously hinder people's physiological and psychological comfort, adult products such as masturbation cups satisfy people's needs to a certain extent.

As one kind of adult products, a masturbation cup is a tool for simulating the female organ from special silica gel to allow males to release their sex pressure. Permitted legally and morally, the masturbation cup well helps males to be sexually satisfied even with the lonely heart.

Although there are a variety of commercially available masturbation cups, their principle is similar. Such conventional masturbation cups are heavy and monotonic, and are difficult to provide visually and audibly intuitive experience in spite of the tactile experience. In terms of interaction, almost no visual feedback is provided to users. Due to such defects in design, better "sexual" experience can't be achieved through masturbation cups.

**SUMMARY OF THE PRESENT INVENTION**

In view of the deficiencies in the prior art, an objective of the present invention is to provide a method and system for interacting with an intelligent adult product. By allowing a user to view a virtual and vivid character scene by wearing a VR box and then controlling the motion of characters in the virtual scene by an intelligent masturbation cup or an intelligent bracelet, the motion synchronization between a real character and a virtual character is realized. Thus, the user is provided with visually and audibly immersive experience, and also most exciting tactile experience.

The objective of the present invention is realized by the following technical solutions.

The present invention provides a method for interacting with an intelligent adult product, including the following steps of:

> establishing connection between an intelligent adult product and an application in a user's mobile terminal;
> controlling, by an acceleration sensor in the intelligent adult product, the motion of a three-dimensional character in the application; and
> viewing, by a user and by using a VR box, sounding persons and objects in a virtual scene in a first-person perspective or a third-person perspective

Further, before establishing connection between an intelligent adult product and an application in a user's mobile terminal, the method further includes the following steps of:

> installing, in an intelligent mobile terminal, an application for presenting a character model and a motion animation established by 3dmax molding; and
> rendering, by the application, 3D left-eye and right-eye pictures with a parallax in real time.

Further, the intelligent adult product is an intelligent masturbation cup or an intelligent bracelet.

Further, the step of controlling, by an acceleration sensor in the intelligent adult product, the motion of a three-dimensional character in the application further includes:

> performing low-pass filtering on original acceleration data;
> normalizing the low-pass filtered acceleration data; and
> smoothing the normalized data, and then transmitting the smoothed data to the three-dimensional character.

Further, low-pass filtering is performed on the original acceleration data by a multi-order low-pass filtering algorithm, and a formula for low-pass filtering is as follows:

$$Y_n = \sum_{i=0}^{n} b_i X_{n-i} - \sum_{j=1}^{n} a_j Y_{n-j}$$

where $X_{n-i}$ is the original acceleration data, $Y_n$ is the filtered acceleration data, $a_j$ and $b_i$ are filtering coefficients, i,j denotes an order index of the multi-order low-pass filtering algorithm, and n denotes the order of the algorithm.

Further, the low-pass filtered acceleration data is normalized as a floating-point number from 0 to 1, an interval from a maximum value maxAccel to a minimum value minAccel in a data queue is used as a quantized interval for normalization, and the normalized depth value Depth is calculated according to the following formula:

$$Depth = \frac{curAccel - minAccel}{maxAccel - minAccel}$$

where curAccel is the acceleration value at the current moment, Depth is the normalized depth value, and the normalized depth value Depth is used for controlling the motion of the three-dimensional character; and, when maxAccel is equal to minAccel, it is indicated that the intelligent adult product is in a static state, and Depth is set to a default value 0.

Further, the smoothing is calculated by the following formula:

$$Depth = LastDepth \cdot (1 - \alpha) + ExpDepth \cdot \alpha$$

where $\alpha$ is a smoothing coefficient, ExpDepth is a desired depth before each update, and LastDepth is a historical depth represented by the current motion position of the three-dimensional character.

Further, the sound includes the type of the sound and the volume of the sound, and is controlled by the frequency of the acceleration sensor in the intelligent adult product.

The present invention further provides a system for interacting with an intelligent adult product, including:

a connection establishment module configured to establish connection between an intelligent adult product and an application in a user's mobile terminal;

a control module configured to control, by an acceleration sensor in the intelligent adult product, the motion of a three-dimensional character in the application; and

a VR box viewing module configured to be used by a user, by using a VR box, to view sounding persons and objects in a virtual scene in a first-person perspective or a third-person perspective.

Further, an installation module is configured to: before establishing a connection between an intelligent adult product and an application in a user's mobile terminal, install, in the intelligent mobile terminal, an application for presenting a character model and a motion animation established by 3dmax molding; and the application renders 3D left-eye and right-eye pictures with a parallax in real time.

Compared with the closest prior art, the technical solutions provided by the present invention has the following beneficial effects.

In the present invention, pictures in the first-person or third-person perspective are provided for a user by the VR technology, so that the user is provided with visually, tactilely and audibly immersive experience. In this design, by controlling the motion of a three-dimensional character by an acceleration sensor, the cost in production and application can be greatly reduced. During controlling the motion of the three-dimensional character by the acceleration, it is required to first perform low-pass filtering on acceleration data, then normalize the acceleration data as a floating-point number from 0 to 1, subsequently smooth the numerical value once, and finally transmit the data to the three-dimensional character. The motion animation of the three-dimensional character is also to be normalized between 0 and 1, which exactly corresponds to the normalized data of the acceleration. Thus, the whole motion amplitude from, by a user, operating an intelligent masturbation cup or an intelligent bracelet to viewing the motion of the three-dimensional character is synchronized well. Meanwhile, the sound of the character in the three-dimensional scene is also controlled by the frequency of the intelligent masturbation cup or the intelligent bracelet.

Compared with the conventional adult products, by the masturbation cup of the present invention, the user may be provided with excellent visual, tactile and audible experience, and the user may interact with the three-dimensional character visually by the intelligent masturbation cup or the intelligent bracelet while viewing the three-dimensional effect, so that the interestingness is enhanced. In the present invention, both the intelligent masturbation cup and the intelligent bracelet are provided with an acceleration sensor, so that the production cost is greatly reduced in comparison to some expensive sensors.

**BRIEF DESCRIPTION OF THE DRAWINGS**

To describe the technical solutions in the embodiments of the present invention or in the prior art more clearly, the accompanying drawings to be used in the descriptions of the embodiments or the prior art will be briefly described below. Apparently, the accompanying drawings described hereinafter are some of embodiments of the present invention, and a person of ordinary skill in the art can obtain other drawings according to these drawings without any creative effort.

Fig. 1 is a principle diagram of a method for interacting with an intelligent adult product;
Fig. 2 is a comparison diagram of acceleration data, where (a) denotes original data and (b) denotes the filtered data; and
Fig. 3 is a structural block diagram of a method for interacting with an intelligent adult product.

**DETAILED DESCRIPTION OF THE PRESENT INVENTION**

To make the objectives, technical solutions and advantages of the present invention clearer, the technical solutions of the present invention will be described below in detail. Apparently, the embodiments described herein are a part of but not all of the embodiments of the present invention. All other implementations obtained by one of ordinary skill in the art without any creative labor on the basis of the embodiments in the present invention shall fall into the protection scope of the present invention.

Embodiment 1

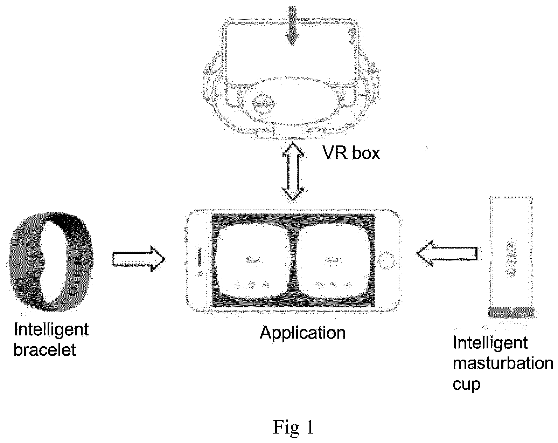

The present invention provides a method for interacting with an intelligent adult product. Fig. 1 shows the principle of the method of the present invention. Before the connection between the intelligent adult product and an application in a user's intelligent mobile terminal is established, a customized application is installed in the mobile phone, and the software presents a character model and a motion animation established by 3dmax molding. The software renders left-eye and right-eye pictures with a parallax in real time by a virtual binocular camera (a virtual VR box). Due to the gyroscope data of the mobile phone, a user may view the 3D stereoscopic effect by wearing the VR box. The intelligent adult product is an intelligent masturbation cup or an intelligent bracelet.

The connection between the intelligent masturbation cup or the intelligent bracelet and the application is established by Bluetooth, and the data from the acceleration sensor is converted into a floating-point number within an interval from 0 to 1. A character model and a three-dimensional scene are established in a realistic style by 3dmax, and a motion animation is provided for the three-dimensional character. The position of the motion animation of the three-dimensional character is normalized between 0 and 1. This makes preparations for Bluetooth control. A corresponding sound file will be played during the motion of the character, and the type of the sound and the volume of the sound are controlled by the frequency of the sensor data.

The method specifically includes the following steps.

S1: The connection between an intelligent adult product and an application in a user's mobile terminal is established, including: establishing connection between an intelligent masturbation cup or an intelligent bracelet and the application in the user's intelligent mobile terminal through Bluetooth;
S2: The motion of a three-dimensional character in the application is controlled by an acceleration sensor in the intelligent adult product, including the following steps.

S201: Low-pass filtering is performed on original acceleration data.

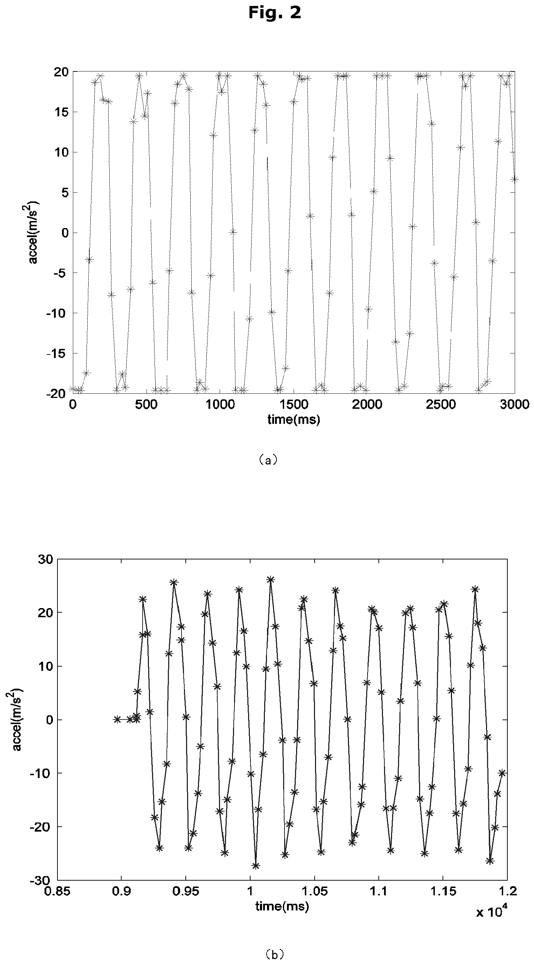

Fig. 2 is a comparison diagram before and after filtering the sensor data. Since the periodic motion of an electric motor of the intelligent masturbation cup results in many noise points, it is difficult to determine peaks and troughs, and the smoothing of the animation of the character is also changed suddenly. In the present invention, a multi-order low-pass filtering algorithm is used to smooth the original acceleration data, and the smoothed data has clearer peaks and troughs. A formula for low-pass filtering is as follows:

$$Y_n = \sum_{i=0}^{n} b_i X_{n-i} - \sum_{j=1}^{n} a_j Y_{n-j}$$

where $X_{n-i}$ is the original acceleration data, $Y_n$ is the filtered acceleration data, $a_j$ and $b_i$ are filtering coefficients, i,j denotes an order index of the multi-order low-pass filtering algorithm, and n denotes the order of the algorithm. In the present invention, 10-level filtering is adopted, and all filtering coefficients are generated by matlab.

S202: The low-pass filtered acceleration data is normalized.

In the present invention, in the algorithm for converting the acceleration data into a floating-point number from 0 to 1, two queues are specifically formed to store the original acceleration data and the filtered acceleration data, respectively, and the size of the queues is limited. When the queues are full, an old data is removed from the queues, and a new data will be added to the queues. In the queue of the filtered data, a maximum value macAccel and a minimum value minAccel are found continuously as a quantized interval for normalization. Thus, a formula is provided as follows:

$$Depth = \frac{curAccel - minAccel}{maxAccel - minAccel}$$

where curAccel is the acceleration value at the current moment, Depth is the normalized depth value, and the normalized depth value Depth is used for controlling the motion of the three-dimensional character. When maxAccel is equal to minAccel, it is indicated that the intelligent masturbation cup or intelligent bracelet is in a static state, and Depth may be set to a default value 0. By statistically recording the Depth data, the to-and-fro frequency Freq of the intelligent masturbation cup or bracelet may be calculated. When the value of Freq is greater than a certain value, a rushing sound file may be played at a larger volume. When the value of Freq is less than or equal to a certain value, a gentle sound file may be played at a smaller volume.

S203: The normalized data is smoothed, and then the smoothed data is transmitted to the three-dimensional character.

The to-and-fro motion of the three-dimensional character may be controlled by the acquired data Depth. However, unsmooth effects such as jittering will be caused only by using the above data to control the motion of the character, because the rendering frame rate of the three-dimensional scene is different from the frequency of the receiving sensor of the application. To make the motion of the character smoother, this design provides a smoothing algorithm.

$$Depth = LastDepth \cdot (1 - \alpha) + ExpDepth \cdot \alpha$$

Before each rendering of the three-dimensional scene, the motion of the three-dimensional character will be updated once, that is, the Depth will be updated once. By using the normalized Depth of the sensor as a desired depth ExpDepth before each update and the current motion position of the three-dimensional character as a historical depth LastDepth, the Depth calculated according to the formula is the final position of the motion of the character. In the formula, $\alpha$ is a smoothing coefficient. Through lots of experiments, it is determined that the experience value of $\alpha$ is 0.65.

S3: The user views, by using a VR box, sounding persons and objects in a virtual scene in a first-person perspective or a third-person perspective.

A corresponding sound file will be played during the motion of the three-dimensional character, and the type of the sound and the volume of the sound are controlled by the frequency of the sensor data. The application is activated, the mobile phone is inserted into the VR box with the middle vertical line aligned with the VR box, and the VR box is worn on the user's head. The user views persons and objects in the virtual scene in a first-person perspective as the three-dimensional character or in a third-person perspective. When the user turns his head, the pictures viewed by the user are also updated, and the user can control the background music and brightness of the scene and perform other operations according to the holding duration of a button in the focused scene. This 360-degree viewing and operation manner can provide the user with strong sense of immediacy.

Embodiment 2

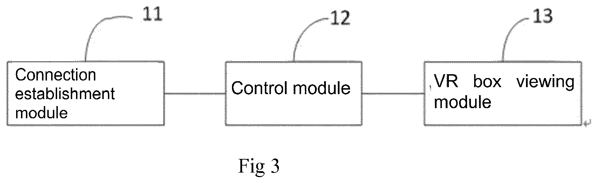

Based on the same inventive idea, the present invention further provides a system for interacting an intelligent adult product. Fig. 3 shows a structural diagram of this system. This system includes:

a connection establishment module 11 configured to establish connection between an intelligent adult product and an application in a user's mobile terminal;

a control module 12 configured to control, by an acceleration sensor in the intelligent adult product, the motion of a three-dimensional character in the application; and

a VR box viewing module 13 configured to be used by a user, by using a VR box, to view sounding persons and objects in a virtual scene in a first-person perspective or a third-person perspective.

The system further includes an installation module which is configured to: before establishing a connection between an intelligent adult product and an application in a user's mobile terminal, install, in the intelligent mobile terminal, an application for presenting a character model and a motion animation established by 3dmax molding; the application renders 3D left-eye and right-eye pictures with a parallax in real time; and a user views, by wearing a VR box, the stereoscopic effect in real time.

The present invention provides an intelligent virtual sex solution. By allowing a user to view a virtual and vivid character scene by wearing a VR box and then controlling the motion of characters in the virtual scene by an intelligent masturbation cup or an intelligent bracelet, the motion synchronization between a real character and a virtual character is realized. Thus, the user is provided with visually and audibly immersive experience, and also most exciting tactile experience.

The foregoing descriptions merely show specific implementations of the present invention, but the protection scope of the present invention is not limited thereto. All variations or replacements which may be easily conceived by a person of skill in the art within the technical scope disclosed by the present invention shall fall into the protection scope of the present invention. Accordingly, the protection scope of the present invention shall be subject to the protection scope of the claims.

**Claims**

1. A method for interacting with an intelligent adult product, comprising the following steps of:

establishing connection between an intelligent adult product and an application in a user's mobile terminal;
controlling, by an acceleration sensor in the intelligent adult product, the motion of a three-dimensional character in the application; and
viewing, by a user and by using a VR box, sounding persons and objects in a virtual scene in a first-person perspective or a third-person perspective.

2. The method for interacting with an intelligent adult product according to claim 1, before establishing connection between an intelligent adult product and an application in a user's mobile terminal, further comprising the following steps of:

installing, in an intelligent mobile terminal, an application for presenting a character model and a motion animation established by 3dmax molding;
rendering, by the application, 3D left-eye and right-eye pictures with a parallax in real time; and
viewing, by a user and by wearing the VR box, the stereoscopic effect in real time.

3. The method for interacting with an intelligent adult product according to any one of claims 1 to 2, **characterized in that** the intelligent adult product is an intelligent masturbation cup or an intelligent bracelet.

4. The method for interacting with an intelligent adult product according to claim 1, **characterized in that** the step of controlling, by an acceleration sensor in the intelligent adult product, the motion of a three-dimensional character in the application further comprises:

performing low-pass filtering on original acceleration data;
normalizing the low-pass filtered acceleration data; and
smoothing the normalized data, and then transmitting the smoothed data to the three-dimensional character.

5. The method for interacting with an intelligent adult product according to claim 4, **characterized in that** low-pass filtering is performed on the original acceleration data by a multi-order low-pass filtering algorithm, and a formula for low-pass filtering is as follows:

$$Y_n = \sum_{i=0}^{n} b_i X_{n-i} - \sum_{j=1}^{n} a_j Y_{n-j}$$

where $X_{n-i}$ is the original acceleration data, $Y_n$ is the filtered acceleration data, $a_j$ and $b_i$ are filtering coefficients, i,j denotes an order index of the multi-order low-pass filtering algorithm, and n denotes the order of the algorithm.

6. The method for interacting with an intelligent adult product according to claim 4, **characterized in that** the low-pass filtered acceleration data is normalized as a floating-point number from 0 to 1, an interval from a maximum value maxAccel to a minimum value minAccel in a data queue is used as a quantized interval for normalization, and the normalized depth value Depth is calculated according to the following formula:

$$Depth = \frac{curAccel - minAccel}{maxAccel - minAccel}$$

where curAccel is the acceleration value at the current moment, Depth is the normalized depth value, and the normalized depth value Depth is used for controlling the motion of the three-dimensional character; and, when maxAccel is equal to minAccel, it is indicated that the intelligent adult product is in a static state, and Depth is set to a default value 0.

7. The method for interacting with an intelligent adult product according to claim 4, **characterized in that** the smoothing is calculated by the following formula:

$$Depth = LastDepth \cdot (1 - \alpha) + ExpDepth \cdot \alpha$$

where $\alpha$ is a smoothing coefficient, ExpDepth is a desired depth before each update, and LastDepth is a historical depth represented by the current motion position of the three-dimensional character.

8. The method for interacting with an intelligent adult product according to claim 1, **characterized in that** the sound comprises the type of the sound and the volume of the sound, and is controlled by the frequency of the acceleration sensor in the intelligent adult product.

9. A system for interacting with an intelligent adult product, comprising:

a connection establishment module configured to establish connection between an intelligent adult product and an application in a user's mobile terminal;
a control module configured to control, by an acceleration sensor in the intelligent adult product, the motion of a three-dimensional character in the application; and
a VR box viewing module configured to be used by a user, by using a VR box, to view sounding persons and objects in a virtual scene in a first-person perspective or a third-person perspective.

10. The system for interacting with an intelligent adult product according to claim 9, **characterized in that** an installation module is configured to: before establishing a connection between an intelligent adult product and an application in a user's mobile terminal, install, in the intelligent mobile terminal, an application for presenting a character model and a motion animation established by 3dmax molding; the application renders 3D left-eye and right-eye pictures with a parallax in real time; and a user views, by wearing a VR box, the stereoscopic effect in real time.

VR box

Intelligent
bracelet

Application

Intelligent
masturbation
cup

Fig 1

# Fig. 2

(a)

(b)

Fig 3

Europäisches
Patentamt

European
Patent Office

Office européen
des brevets

# EUROPEAN SEARCH REPORT

Application Number

EP 17 17 6367

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/260262 A1 (BAKER JOHN [US] ET AL) 8 September 2016 (2016-09-08) | 1-3,8-10 | INV. G06F3/01 A61H19/00 G06F3/0346 |
| Y | * paragraphs [0002] - [0101]; figures 1-5 * | 4-7 | |
| X | US 2006/079732 A1 (BLUMENTHAL YACOB [IL]) 13 April 2006 (2006-04-13) | 1-3,8-10 | |
| Y | * paragraphs [0001] - [0069]; figures 1-8 * | 4-7 | |
| Y | US 2002/103610 A1 (BACHMANN ERIC R [US] ET AL) 1 August 2002 (2002-08-01) * paragraphs [0002] - [0110]; figures 1-6 * | 4-7 | |
| T | Anonymous: "New Oculus Rift dev kit uses the front of a Galaxy Note 3 as its screen - The Verge", 31 July 2014 (2014-07-31), XP055446961, Retrieved from the Internet: URL:https://www.theverge.com/2014/7/31/5956589/new-oculus-dev-kit-uses-front-of-galaxy-note-3-for-display [retrieved on 2018-02-01] * the whole document * | | |
| T | Anonymous: "Samsung Gear VR - Wikipedia", 18 January 2018 (2018-01-18), XP055447218, Retrieved from the Internet: URL:https://en.wikipedia.org/wiki/Samsung_Gear_VR [retrieved on 2018-02-02] * the whole document * | | TECHNICAL FIELDS SEARCHED (IPC) G06F A61H |

-/--

The present search report has been drawn up for all claims

| Place of search | Date of completion of the search | Examiner |
|---|---|---|
| Munich | 5 February 2018 | Quesson, Christophe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**Europäisches Patentamt**
**European Patent Office**
**Office européen des brevets**

# EUROPEAN SEARCH REPORT

Application Number

EP 17 17 6367

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Anonymous ET AL: "Oculus Rift Best Practices", , 31 December 2015 (2015-12-31), XP055447475, Retrieved from the Internet: URL:http://static.oculus.com/sdk-downloads /documents/Oculus_Best_Practices_Guide_0.5 .0.pdf [retrieved on 2018-02-02] * the whole document * ----- | | |
| T | Anonymous: "Trademarks", , 1 February 2018 (2018-02-01), XP055447433, Retrieved from the Internet: URL:https://www.autodesk.com/company/trade markblock [retrieved on 2018-02-02] ----- | | |

TECHNICAL FIELDS
SEARCHED      (IPC)

The present search report has been drawn up for all claims

| Place of search | Date of completion of the search | Examiner |
|---|---|---|
| Munich | 5 February 2018 | Quesson, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 6367

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2018

| Patent document cited in search report | | Publication date | Patent family member(s) | | Publication date |
|---|---|---|---|---|---|
| US 2016260262 | A1 | 08-09-2016 | CA | 2979036 A1 | 15-09-2016 |
| | | | EP | 3267961 A1 | 17-01-2018 |
| | | | US | 2016260262 A1 | 08-09-2016 |
| | | | WO | 2016144948 A1 | 15-09-2016 |
| US 2006079732 | A1 | 13-04-2006 | NONE | | |
| US 2002103610 | A1 | 01-08-2002 | AU | 3057802 A | 15-05-2002 |
| | | | US | 7089148 B1 | 08-08-2006 |
| | | | US | 2002103610 A1 | 01-08-2002 |
| | | | WO | 0237827 A2 | 10-05-2002 |

EPO FORM P0459

For more details about this annex : see Official Journal of the European Patent Office, No. 12/82